# EUROPEAN PATENT APPLICATION

(11) **EP 3 919 091 A1**
(43) Date of publication of application: **08.12.2021**
(21) Application number: 20178474.1
(22) Date of filing: 05.06.2020
(51) Int. Cl.: A61L 27/26, A61L 27/52, B33Y 70/00

(54) **BIOINK FOR REVERSIBLY FORMING A HYDROGEL BY LIGHT**

(71) Applicant: Albert-Ludwigs-Universität Freiburg, 79098 Freiburg (DE)
(72) Inventor: WEBER, Wilfried, 79100 Freiburg (DE); HÖRNER, Maximilian, 79108 Freiburg (DE)
(74) Representative: Ter Meer Steinmeister & Partner

(57) **Abstract**

The present bioink comprises a first polymer coupled to a first protein and a second polymer coupled to a second protein, wherein the first protein and the second protein form a heterodimer upon irradiation with visible or infrared light in a specific wavelength range, which can be reversed upon irradiating the heterodimer with visible or infrared light in a second wavelength range, wherein the second wavelength range is different from the first wavelength range. The bioink enables a quick and reversible formation of hydrogels using cell-friendly light.

## Description

The present invention relates to a novel bioink, a method for reversibly forming a hydrogel with the bioink of the present invention and to a hydrogel. The present invention also relates to a cartridge for a 3D printer comprising the bioink of the present invention.

### Background of the invention

Current light-responsive matrices for 3D printing require high doses of high-energy light (in particular UV light) for hardening. Therefore, compromises have to be found between a sufficiently short hardening time and a light dose low enough not to harm cells. Further, the current light-responsive matrices are irreversibly cross-linked which poses two problems: First, it is impossible or at least very complicated to remove or dissolve the matrix, for example in order to release cells for subsequent analysis when used in drug testing. Second, when printing tissues it is important to remove the artificial matrix, in order to give the cells space for growth and differentiation and not to impair integration with the target tissue in patients. Further, current bioinks are static and cannot be dynamically modulated with regard to mechanical properties. This makes it impossible to adjust bioink mechanics to cell differentiation needs.

WO 2019/195256 A1 is directed to hydrogel compositions comprising a triblock copolymer having a formula A-B-A, wherein A is a polycaprolactone (PCL) block or a polyvalerolactone (PVL) block and B is a polyethylene glycol (PEG) block. Also disclosed are methods of making a hydrogel comprising providing a photoinitiator and a triblock copolymer having a formula A-B-A, wherein the triblock copolymer comprises one or more ethylenically unsaturated moieties, and photocrosslinking the triblock copolymer, thereby forming a hydrogel. The formation of the hydrogel is however irreversible, and its mechanical properties cannot be modulated over time.

Horner et al. (Adv. Mater. 2019, 31, 1806727) discloses a phytochrome-based extracellular matrix with tuneable mechanical properties using light of different wavelength. However, the phytochrome-based extracellular matrix has a long gelation time of 20 h. Further, the matrix cannot be completely dissolved and remains in a solid state.

### Disclosure of Invention

### Technical Problem

Hence, it was an object of the present invention to overcome the above-mentioned disadvantages.

It was an object of the present invention to provide a bioink which can be solidified by low doses of cell friendly light, in particular not UV.

It was a further object of the present invention to provide a liquid bioink which, once gelated, can also be dissolved by low doses of cell friendly light, in particular not UV, thus re-obtaining the liquid bioink.

It was an object of the present invention to provide a bioink from which individual cells can be extracted by locally liquifying the surrounding solidified bioink via low doses of cell friendly light, in particular not UV.

It was an object of the present invention to provide a bioink whose mechanical properties, once solidified, can be modulated over time by the simple illumination with a selected wavelength range in the red or near-infrared range.

It was a further object of the present invention to provide a bioink with a short gelation time.

It was an object of the present invention to provide a bioink which can contain living cells during hydrogel formation.

It was a further object of the present invention to provide a bioink with reduced cross-linking before hydrogel formation.

It was a further object of the present invention to provide a bioink that is stable, i.e. does not form a hydrogel, when it is not irradiated with light in a specific wavelength range.

### Solution to Problem

All these objects are surprisingly solved by a bioink of the present invention.

In an aspect of the present invention, the bioink comprises a first polymer coupled to a first protein, a second polymer coupled to a second protein, wherein the first protein and the second protein form a heterodimer upon irradiation with visible or infrared light in a first wavelength range, which can be reversed upon irradiating the heterodimer with visible or infrared light in a second wavelength range, wherein the second wavelength range is different from the first wavelength range.

In a further aspect of the present invention, a cartridge for a 3D printer containing the bioink is provided.

In another aspect of the present invention, a method for reversibly preparing a hydrogel is provided, which includes the steps of: (i) preparing a bioink of the present invention; (ii) printing the bioink, wherein printing comprises irradiating the bioink with visible or infrared light in a first wavelength range to obtain a hydrogel.

In an embodiment, the method further comprises (iii) irradiating the hydrogel with visible or infrared light in a specific second wavelength range to dissolve the hydrogel, wherein the second wavelength range of the light in step (iii) is different from the first wavelength range of the light in step (ii).

In still another aspect of the present invention, a hydrogel formed by irradiating the bioink with visible or infrared light in a first wavelength range is provided.

### Advantageous Effects of Invention

Since bioinks according to various exemplary embodiments of the present invention comprise a first polymer coupled to a first protein and a second polymer coupled to a second protein, wherein the first protein and the second protein reversibly form a heterodimer upon irradiation with visible or infrared light in a specific wavelength range, the bioink can be readily cross-linked, thus forming a hydrogel.

Further, since the heterodimer formation is reversible using visible or infrared light in a specific wavelength range, which is different from the wavelength range for heterodimer formation, the hydrogel can also be readily dissolved.

Using two separate polymers coupled to two different proteins which can form a heterodimer ensures that cross-linking occurs between different polymers and not within the same polymer, which is likely to occur if proteins are used which form homodimers, e.g. if a polymer is coupled to more than one protein.

Since hydrogel formation can only occur by irradiating the bioink with visible or infrared light in a specific wavelength range, the bioink is stable for a long period of time, if not irradiated.

Since hydrogel formation occurs under mild conditions, the bioink can contain living cells during hydrogel formation.

Further, since the hydrogel can be dissolved quickly and under mild conditions, cells can be easily extracted from the hydrogel.

Additionally, the resulting bioink can be applied to various fields including hydrogel fields such as a tissue-engineered scaffold or as drug depot in drug screenings.

### Brief Description of the Figures

**Fig. 1** is a schematic representation of the bioink according to the invention.
**Fig. 2** reproduces nucleic acid sequences of described plasmids. The open reading frames are underlined. a) plasmid pMH610; b) plasmid pMH613; c) plasmid pMH620; d) plasmid pMH623.
**Fig. 3** shows a microfluidic chip in which the bioink of the present invention was polymerized spatially resolved using 660 nm light and the depicted photomask.
**Fig. 4** shows spatially resolved deposition of mammalian cells in a microfluidic chip using the bioink.
**Fig. 5** shows the results of a small amplitude oscillatory shear rheology experiment with the bioink of the invention.

### Detailed description of the invention

The bioink of the present invention comprises a first polymer coupled to a first protein and a second polymer coupled to a second protein, wherein the first protein and the second protein reversibly form a heterodimer upon irradiation with visible or infrared light in a specific wavelength range. Specifically, the first protein and the second protein reversibly form a heterodimer upon irradiation with visible or infrared light in a first wavelength range. The heterodimer can be dissolved by irradiation with visible or infrared light in a second wavelength range, wherein the first and the second wavelengths ranges are different.

"Visible light" as used herein excludes light having a wavelength smaller than 400 nm (i.e. UV light). Visible light has a wavelength in the range of range 400 to less than 700 nm. "Infrared light" as used herein refers to light having a wavelength in the range of 700-1000 nm. "Near-infrared light" as used herein refers to light in a wavelength range of 700-790 nm.

As used herein, "a first polymer coupled to a first protein" does not mean that a first polymer is coupled only to a single protein. On the contrary, the first polymer can be coupled to more than one protein. However, all the proteins are a first protein, i.e. they are all identical. Similarly, "a second polymer coupled to a second protein" does not exclude that the second polymer is coupled to more than one (second) protein. However, all the proteins are a second protein, i.e. they are all identical. In fact, the terms "a first polymer coupled to a first protein" and "a second polymer coupled to a second protein" are synonymous with "a first polymer coupled to at least a first protein" and "a second polymer coupled to at least a second protein".

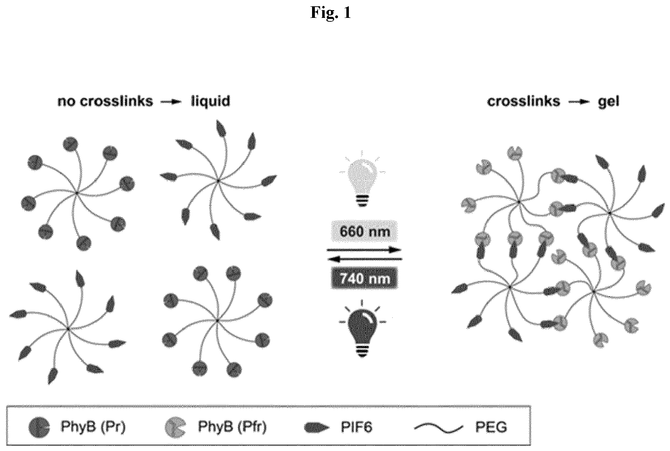

A schematic representation of an exemplary bioink of the present invention is shown in Fig. 1. A first polymer (here: 8-arm PEG) is coupled to a first protein (here: PhyB), specifically each PEG molecule is coupled to 8 PhyB molecules, and a second polymer (here: also 8-arm PEG) is coupled to a second protein (here: PIF6), specifically each PEG molecule is coupled to 8 PIF6. When a mixture of first and second polymer is irradiated with visible light with a wavelength of about 660 nm, PhyB is activated and the activated PhyB interacts with PIF6 to form a heterodimer. By the heterodimerization, cross-links are formed between the first and second polymers, leading to the formation of a hydrogel. The hydrogel can be then dissolved by irradiating it with infrared light with a wavelength of about 740 nm. Hereby, the activated state of PhyB is reversed. The ground state of PhyB cannot interact with PIF6, so that heterodimerization is reversed, the cross-links between the first and second polymers are thus dissolved, and so is the hydrogel. Hence, the liquid bioink is re-obtained which can be cross-linked again by irradiating it with visible light with a wavelength of about 660 nm. Gel formation and gel dissolution is thus a reversible process with the bioink of the present invention.

For use in the present invention, any first and second proteins which can reversibly form a heterodimer upon irradiation with visible or infrared light can be used. Specifically, heterodimer forming proteins suitable for the present invention are those that upon irradiation with visible or infrared light in a first wavelength range form a heterodimer, wherein the heterodimerization is reversed by irradiating the heterodimer with visible or infrared light in a second wavelength range. In an embodiment, a first protein suitable for the present invention has an absorbance maximum of the ground state within a narrow wavelength range which leads to a rapid conformational change to form an activated state. The first protein in the activated state is then able to form a heterodimer with the second protein. The activated state itself has an absorbance maximum (different from the absorbance maximum of the ground state) which converts the activated state of the first protein back to the ground state. Since the ground state of the first protein cannot form a heterodimer with the second protein, heterodimerization is reversed.

In the present invention. the first wavelength range and the second wavelength range are different. Two wavelength ranges are regarded as different not only if the wavelength ranges of the two wavelength ranges are different, but also if - given the same wavelength range - the intensities of the wavelengths within the wavelength ranges are different. For example, the first and second wavelength ranges may be 650 to 750 nm. The first wavelength range may have a peak wavelength (wavelength of highest intensity) at 660 nm, the second wavelength range may have a peak wavelength at 740 nm. According to the above definition, these first and second wavelength ranges are different.

In one embodiment, the first wavelength range includes the wavelength of the absorbance maximum of the ground state which converts the ground state of the first protein to its activated state, but it does not include the wavelength of the absorbance maximum of the activated state which converts the activated state of the first protein to its ground state. Preferably, the wavelength of the absorbance maximum of the ground state of the first protein has the highest intensity in the first wavelength range. Accordingly, the second wavelength range includes the wavelength of the absorbance maximum of the activated state which converts the activated state of the first protein to its ground state, but it does not include the wavelength of the absorbance maximum of the ground state which converts the ground state of the first protein to its activated state. Preferably, the wavelength of the absorbance maximum of the activated state of the first protein has the highest intensity in the second wavelength range.

In another embodiment, which is less preferred, the first wavelength range includes the wavelength of absorbance maximum of the ground state which converts the ground state of the protein to its activated state, and it also includes the wavelength of absorbance maximum of the activated state which converts the activated state of the protein to its ground state. However, the intensity of the wavelength of the absorbance maximum of the ground state is higher than the intensity of the wavelength of the absorbance maximum of the activated state. Accordingly, the second wavelength range includes the wavelength of absorbance maximum of the activated state which converts the activated state of the protein to its ground state, and it also includes the wavelength of absorbance maximum of the ground state which converts the ground state of the protein to its activated state. However, the intensity of the wavelength of the absorbance maximum of the activated state is higher than the intensity of the wavelength of the absorbance maximum of the ground state. Hence, in an embodiment of the invention, the first and second wavelength ranges can be identical from the point of view of the wavelengths, but are different from the point of view of the intensity of each wavelength. In an embodiment, the first wavelength range is in the visible light range (400 to less than 700 nm) or in the infrared range (700-1000 nm), in particular in the near-infrared range (700-790 nm). The second wavelength range is in the visible light range (400 to less than 700 nm) or in the infrared range (700-1000 nm), in particular in the near-infrared range (700-790 nm). In an embodiment of the present invention, if the first wavelength range is in the visible light range (400 to less than 700 nm), the second wavelength range is preferably in the infrared range(700-1000 nm), in particular in the near-infrared range (700-790 nm). Accordingly, if the first wavelength range is in the infrared range (700-1000 nm), in particular in the near-infrared range (700-790 nm), the second wavelength range is preferably in the visible light range (400 to less than 700 nm).

First and second proteins to be used in the present invention are those who can heterodimerize fast, i.e. in less than 5 min. Heterodimerization can be easily detected using known methods, such as analytical ultracentrifugation or size-exclusion chromatography (SEC)

In an embodiment of the invention, the first protein is a phytochrome and the second protein is a phytochrome interacting partner. The phytochrome and the phytochrome interacting partner are not limited to the wild type, but also include a derivative thereof. A derivative of the phytochrome and phytochrome interacting partner contains mutations or deletions. "Deletion" as used herein means that at least one amino acid of the natural phytochrome is deleted. In one embodiment, the deletion may comprise 0.01% to 98% of the natural phytochrome or phytochrome interacting partner amino acid sequence. For example, a deletion of a phytochrome may be represented by the use of the photosensory module. Mutation as used herein means that at least one amino acid of the wild-type phytochrome is replaced by at least another amino acid. In one embodiment, the mutation may regard up to 3% of the natural phytochrome amino acid sequence.

Phytochromes are a class of photoreceptors in plants, bacteria and fungi used to detect light. They are sensitive to light in the red and far-red region of the visible spectrum and can be classed as either Type I, which are activated by far-red light, or Type II that are activated by red light. Phytochromes are characterized by a red/far-red photochromicity. Photochromic pigments change their "colour" (spectral absorbance properties) upon light absorption. In the case of phytochrome the ground state is Pr, the r indicating that it absorbs red light particularly strongly. The absorbance maximum is in most phytochromes a sharp peak 650-670 nm, so concentrated phytochrome solutions look turquoise-blue to the human eye. But once a red photon has been absorbed, the pigment undergoes a rapid conformational change to form the Pfr state. Here fr indicates that now not red but far-red (also called "near infra-red"; 700-790 nm) is preferentially absorbed. This shift in absorbance is apparent to the human eye as a slightly more greenish colour. When Pfr absorbs far-red light it is converted back to Pr. Hence, red light makes Pfr, far-red light makes Pr. In plants, at least Pfr is the physiologically active or "signalling" state.

Chemically, phytochrome consists of a chromophore, a single bilin molecule consisting of an open chain of four pyrrole rings, covalently bonded to the protein moiety via a highly conserved cysteine amino acid. It is the chromophore that absorbs light, and as a result changes the conformation of bilin and subsequently that of the attached protein, changing it from one state or isoform to the other. The phytochrome chromophore can be phytochromobilin or phycocyanobilin. Bilins are derived from the closed tetrapyrrole ring of haem by an oxidative reaction catalyzed by haem oxygenase to yield their characteristic open chain. Phytochrome interaction partners are proteins that can form heterodimers with the phytochromes. Generally, phytochrome interaction partners can form a heterodimer only with either the Pfr or the Pr form of the phytochrome.

In a further embodiment of the invention, the phytochrome is selected from the group comprising PhyA, PhyB, PhyC, PhyD, PhyE, BphP1 and the phytochrome interacting partner is selected from the group comprising PIF1, PIF2, PIF3, PIF4, PIF5, PIF6, PIF7, PIF8, FHY1, FHL, PpsR2, Q-PAS1.

In a further embodiment of the invention, the phytochrome is selected from the group consisting of PhyA, PhyB, PhyC, PhyD, PhyE, BphP1 and the phytochrome interacting partner is selected from the group consisting of PIF1, PIF2, PIF3, PIF4, PIF5, PIF6, PIF7, PIF8, FHY1, FHL, PpsR2, Q-PAS1.

In a preferred embodiment of the invention, the phytochrome is selected from the group consisting of PhyA, PhyB and BphP1 and the phytochrome interacting partner is selected from the group consisting of PIF3, PIF6, FHY1, FHL, PpsR2 and Q-PAS1.

As used herewith, PhyA, PhyB, PhyC, PhyD, PhyE, BphP1, PIF1, PIF2, PIF3, PIF4, PIF5, PIF6, PIF7, PIF8, FHY1, FHL, PpsR2 and Q-PAS1 are not limited to the wild type, but also include a derivative thereof. A derivative of the listed phytochromes and phytochrome interacting partners contains mutations or deletions. "Deletion" and "mutation" have been defined above.

PhyA, PhyB, PhyC, PhyD, PhyE are regulatory photoreceptors which exist in two forms that are reversibly interconvertible by light: the Pr form that absorbs maximally in the red region of the spectrum and the Pfr form that absorbs maximally in the far-red region. Photoconversion of Pr to Pfr induces an array of morphogenetic responses, whereas reconversion of Pfr to Pr cancels the induction of those responses. Preferably, PhyA, PhyB, PhyC, PhyD and PhyE are obtained from *Arabidopsis thaliana.* The sequence listings of these proteins can be found in https://www.uniprot.org/uniprot/P14712 (PhyA), https://www.uniprot.org/uniprot/P14713 (PhyB), https://www.uniprot.org/uniprot/P14714 (PhyC), https://www.uniprot.org/uniprot/P42497 (PhyD), https://www.uniprot.org/uniprot/P42498 (PhyE).

BphP1 is a bacterial phytochrome and is preferably obtained from *Rhodopseudomonas palustris.* The sequence listing of this protein can be found in https://www.uniprot.org/uniprot/A0A161I5N6.

PIF1, PIF2, PIF3, PIF4, PIF5, PIF6, PIF7 and PIF8 are phytochrome interacting factors and are preferably obtained from *Arabidopsis thaliana.* The sequence listings of these proteins can be found in https://www.uniprot.org/uniprot/Q8GZM7 (PIF1), https://www.uniprot.org/uniprot/Q8L5W8-1 (PIF2), https://www.uniprot.org/uniprot/O80536 (PIF3), https://www.uniprot.org/uniprot/Q8W2F3 (PIF4), https://www.uniprot.org/uniprot/Q84LH8 (PIF5), https://www.uniprot.org/uniprot/Q8L5W7 (PIF6), https://www.uniprot.org/uniprot/Q570R7 (PIF7), https://www.uniprot.org/uniprot/Q8GZ38-1 (PIF8).

FHY1 (far red elongated hypocotyl 1) and FHL are preferably obtained from *Arabidopsis thaliana.* The sequence listings of these proteins can be found in https://www.uniprot.org/uniprot/Q8S4Q6 (FHY1) and https://www.uniprot.org/uniprot/A8MR65-1 (FHL). FHY1 and FHL, the only close FHY1 homolog in *Arabidopsis,* are small (23 and 20 kDa) plant-specific proteins which contain both functional NLS (nuclear localization signal) and NES (nuclear export signal) sequences. The homology between FHY1 and FHL is restricted to the NLS/NES region in their N-terminal half and to the extreme C-terminus. Although the overall similarity at the amino acid level is quite low (<30% identical amino acids), there is good evidence that FHY1 and FHL are nevertheless functional homologs (Plant Cell Physiol. 47(8): 1023-1034 (2006)).

PpsR2 is a transcriptional repressor and is preferably obtained from *Rhodopseudomonas palustris* (Nature Methods 2016 July; 13(7): 591-597). The sequence listing of this protein can be found in https://www.uniprot.org/uniprot/A0A167KP37. Q-PAS1 is a single-domain 17-kDa protein, which is derived from PpsR2 and lacks domains involved in the PpsR2 oligomerization (Nat Chem Biol. 2017 Jun; 13(6): 633-639). The sequence listing of this protein can be found in https://www.uniprot.org/uniprot/A0A167KP37.

PhyA, PhyB, PhyC, PhyD, PhyE can form heterodimers with PIF1, PIF2, PIF3, PIF4, PIF5, PIF6, PIF7 and PIF8. Both PhyA and PhyB form heterodimers with PIF3, PIF6, FHY1, FHL, leading to heterodimers PhyA/PIF3, PhyA/PIF6, PhyA/FHY1, PhyA/FHL, PhyB/PIF3, PhyB/PIF6, PhyB/FHY1, PhyB/FHL.

BphP1 forms heterodimers with both PpsR2 and Q-PAS1, leading to heterodimers BphP1/PpsR2 and BphP1/Q-PAS1.

In a preferred embodiment, the first protein and the second protein form heterodimers PhyA/PIF3, PhyA/PIF6, PhyA/FHY1, PhyA/FHL, PhyB/PIF3, PhyB/PIF6, PhyB/FHY1, PhyB/FHL, BphP1/PpsR2 and BphP1/Q-PAS1. More preferably, the heterodimers are PhyA/FHY1, PhyA/FHL, PhyB/PIF3, PhyB/PIF6, BphP1/PpsR2 and BphP1/Q-PAS1. Most preferably, the heterodimers are PhyB/PIF3 and PhyB/PIF6.

Heterodimerization occurs by irradiating the first protein and the second protein with visible or infrared light in a first specific wavelength range. Heterodimerization is generally fast (i.e., less than 5 min, but often in the range of seconds) and can be detected using known methods, such as analytical ultracentrifugation or size-exclusion chromatography (SEC). The heterodimerization can then be reversed by irradiating the heterodimer with visible or infrared light in a second specific wavelength range. The first specific wavelength range and the second specific wavelength range are different.

When the first protein is a phytochrome and the second protein is a phytochrome interacting partner, heterodimerization occurs by irradiating the phytochrome and the phytochrome interacting partner with visible or infrared light in a first specific wavelength range, wherein this range preferably includes the wavelength of maximum absorbance of the phytochrome in the ground state. The heterodimerization can then be reversed by irradiating the heterodimer with visible or infrared light in a second specific wavelength range, wherein this range preferably includes the wavelength of maximum absorbance of the phytochrome (activated state) in the heterodimer.

Specifically, when the phytochrome in the ground state is irradiated with a wavelength corresponding to the wavelength of maximum absorbance of the phytochrome in the ground state, the highest activation of the phytochrome, i.e. the highest concentration of phytochrome in the activated state, is observed.

Accordingly, when the phytochrome in the activated state (such as in the heterodimer) is irradiated with a wavelength corresponding to the wavelength of maximum absorbance of the phytochrome in the activated state, the highest concentration of phytochrome in the ground state is obtained. This corresponds to about 100% of the phytochrome.

When the phytochrome in the ground state is irradiated with light having a wavelength between the wavelength of maximum absorbance of the phytochrome in the ground state and the wavelength of maximum absorbance of the phytochrome in the activated state, the concentration of phytochrome in the activated state will be reduced compared to irradiation of the phytochrome in the ground state with light having the wavelength of maximum absorbance of the phytochrome in the ground state. The degree of reduction of activation increases with increasing distance from the wavelength of maximum absorbance of the phytochrome in the ground state. The same applies for the phytochrome in the activated state. When the phytochrome in the activated state is irradiated with light having a wavelength between the wavelength of maximum absorbance of the phytochrome in the ground state and the wavelength of maximum absorbance of the phytochrome in the activated state, the concentration of phytochrome in the ground state will be reduced compared to irradiation of the phytochrome in the activated state with light having the wavelength of maximum absorbance of the phytochrome in the activated state. The degree of reduction of inactivation increases with increasing distance from the wavelength of maximum absorbance of the phytochrome in the activated state. Hence, between the wavelength of maximum absorbance of the phytochrome in the ground state and the wavelength of maximum absorbance of the phytochrome in the activated state, a mixture of phytochrome in the ground state and in the activated state in varying ratios can be obtained. This leads to a different concentration of cross-links in the hydrogel of the present invention.

In one embodiment, the first wavelength range includes the wavelength of absorbance maximum of the ground state which converts the ground state of the phytochrome to its activated state, but it does not include the wavelength of absorbance maximum of the activated state which converts the activated state of the phytochrome to its ground state. Preferably, the wavelength of the wavelength of absorbance maximum of the ground state of the phytochrome has the highest intensity in the first wavelength range. Accordingly, the second wavelength range includes the wavelength of absorbance maximum of the activated state which converts the activated state of the phytochrome to its ground state, but it does not include the wavelength of absorbance maximum of the ground state which converts the ground state of the phytochrome to its activated state. Preferably, the wavelength of the absorbance maximum of the activated state of the phytochrome has the highest intensity in the second wavelength range.

In another embodiment, which is less preferred, the first wavelength range includes the wavelength of absorbance maximum of the ground state which converts the ground state of the phytochrome to its activated state, and it also includes the wavelength of absorbance maximum of the activated state which converts the activated state of the phytochrome to its ground state. However, in the first wavelength range the intensity of the wavelength of the absorbance maximum of the ground state is higher than the intensity of the wavelength of the absorbance maximum of the activated state. Accordingly, the second wavelength range includes the wavelength of absorbance maximum of the activated state which converts the activated state of the phytochrome to its ground state, and it also includes the wavelength of absorbance maximum of the ground state which converts the ground state of the phytochrome to its activated state. However, in the second wavelength range the intensity of the wavelength of the absorbance maximum of the activated state is higher than the intensity of the wavelength of the absorbance maximum of the ground state. Hence, in an embodiment of the invention, the first and second wavelength ranges can be identical from the point of view of the wavelengths, but are different from the point of view of the intensity of each wavelength.

When the phytochrome is selected from PhyA to PhyE, the first wavelength range preferably has the wavelength of 660 nm as peak wavelength.

When the phytochrome is selected from PhyA to PhyE, heterodimerization occurs by irradiating the bioink with light preferably in a wavelength range of 400 to less than 700 nm, more preferably 600-690 nm, even more preferably from 640-680 nm, most preferably 650-670 nm.

When the phytochrome is selected from PhyA to PhyE, the second wavelength range preferably has the wavelength of 740 nm as peak wavelength.

Reversion of the heterodimerization occurs in case of PhyA to PhyE by irradiating the heterodimer with light preferably in a wavelength range of 700-790 nm, more preferably 720-770 nm, even more preferably from 730-750 nm.

When the phytochrome is BphP1, the first wavelength range preferably has the wavelength of 760 nm as peak wavelength.

When the phytochrome is BphP1, heterodimerization occurs by irradiating the bioink with light preferably in a wavelength range of 700-900 nm, more preferably 720-780 nm, even more preferably from 750-770 nm.

When the phytochrome is BphP1, the second wavelength range preferably has the wavelength of 640 nm as peak wavelength.

Reversion of the heterodimerization occurs in case of BphP1 by irradiating the heterodimer with light preferably in a wavelength range of 500-690 nm, more preferably 620-670 nm, even more preferably from 630-650 nm.

Accordingly, PhyA/PIF3, PhyA/PIF6, PhyA/FHY1, PhyA/FHL, PhyB/PIF3, PhyB/PIF6, PhyB/FHY1 and PhyB/FHL heterodimers are formed by irradiating the corresponding monomers with light preferably in a wavelength range having the wavelength of 660 nm as peak wavelength. Preferably, the wavelength range is of 400 to less than 700 nm, more preferably 600-690 nm, even more preferably from 640-680 nm, most preferably 650-670 nm. Heterodimerization is reversed by irradiating the heterodimers with light preferably in a wavelength range having the wavelength of 740 nm as peak wavelength. Preferably, the wavelength range is of 700-790 nm, more preferably 720-770 nm, even more preferably from 730-750 nm.

Further, BphP1/PpsR2 and BphP1/Q-PAS1 heterodimers are formed by irradiating the corresponding monomers with light preferably in a wavelength range having the wavelength of 760 nm as peak wavelength. Preferably, the wavelength range is of 700-900 nm, more preferably 720-780 nm, even more preferably from 750-770 nm. Heterodimerization is reversed by irradiating the heterodimers with light preferably in a wavelength range having the wavelength of 640 nm as peak wavelength. Preferably, the wavelength range is of 500 to less than 700 nm, more preferably 620-670 nm, even more preferably from 630-650 nm.

In one embodiment of the present invention, the first polymer and the second polymer are coupled to the first protein and the second protein, respectively, via at least one covalent interaction or at least one non-covalent interaction. Accordingly, if the first the first polymer and the second polymer are coupled to more than one first protein and more than one second protein, respectively, each first protein and second protein is coupled to the first polymer and the second polymer, respectively, via at least one covalent interaction or at least one non-covalent interaction.

Preferably, the first polymer and the second polymer are coupled to the first protein and the second protein, respectively, via at least one covalent interaction. More preferably, the first polymer and the second polymer are coupled to the first protein and the second protein, respectively, via one single covalent interaction. When the first polymer and the second polymer are coupled to the first protein and the second protein, respectively, via one single covalent interaction, the risk is reduced that during the coupling reaction a protein molecule is coupled to two different polymer molecules, thus forming a covalent cross-link which is irreversible. Such cross-links have a negative impact on the mechanical properties of the bioink and the hydrogel formed therefrom.

The type of covalent interaction is not limited, as long as it enables to couple the first polymer and the second polymer to the first protein and the second protein, respectively. In one embodiment of the invention, the covalent interaction is the result of the reaction between the first polymer comprising a first chemical functional group and the first protein comprising a second chemical functional group which is able to chemically bond with the first chemical functional group. Accordingly, the covalent interaction is the result of the reaction between the second polymer comprising a third chemical functional group and the second protein comprising a fourth chemical functional group which is able to chemically bond with the third chemical functional group. In a preferred embodiment, the first and third functional groups of the first and second polymer are the same. In a further preferred embodiment, also the second and fourth functional groups of the first and second proteins are the same.

In an embodiment of the invention, the first or third chemical functional group are introduced to the first or second polymer by means of a material having a first or third chemical functional group. Here, the first or third chemical functional group may be formed at a branch or end of the backbone of the first or second polymer. Alternatively, the first or third chemical functional group may be formed within a linear first or second polymer.

Specifically, the material having a first or third chemical functional group may be one or more bearing a tetrazine group, an alkyne group, an epoxy group, an acryloyl group, an NHS-ester group, a maleimide group or a vinyl sulfone.

Exemplary materials having a first or third chemical functional group are methyltetrazine-amine, methyltetrazine-PEG4-amine, methyltetrazine-propylamine, tetrazine-PEG5-NHS ester, methyltetrazine-PEG4-NHS ester, methyltetrazine-sulfo-NHS ester, methyltetrazine-PEG4-acid, methyltetrazine- PEG12-NHS ester, methyltetrazine-NHS ester, methyltetrazine-acid, tetrazine-acid, amino-PEG4-alkyne, alkyne-PEG5-acid, alkyne-PEG-amine, oxiranylamine, 2-oxiranyl-ethylamine, acrylamide, acrylic acid, acryloyl chloride and divinyl sulfone.

Therefore, the introduced first or third chemical functional group may be a tetrazine group, an alkyne group, an epoxy group, an acryloyl group, an NHS-ester group, a maleimide group or a vinyl sulfone group.

In an embodiment of the invention, the second or fourth chemical functional group are introduced to the first or second protein by means of a material having a second or fourth chemical functional group. Here, the second or fourth chemical functional group may be formed at a branch, such as at a side chain of an amino acid, or end of the backbone of the first or second protein.

Specifically, the material having a second or fourth chemical functional group may be one or more bearing cyclooctene group, an azide group, a thiol group or an amine group.

Exemplary materials having a second or fourth chemical functional group are trans-cyclooctene-amine, trans-cyclooctene-NHS ester, trans-cyclooctene-PEG-NHS ester, transcyclooctene-PEG4-acid, azide-PEG4-amine, 3-amino-1-ropanethiol, 11-mercaptoundecanoic acid, amino-methanethiol, cysteine, thiol PEG amine, ethylene diamine, PEG diamine, (S)-3-amino-2-(hydroxymethyl)propionic acid and amino-acetic acid. Therefore, the introduced second or fourth chemical functional group may be a cyclooctene group, an azide group, a thiol group or an amine group.

That is, a combination of the first or third chemical functional group and the second or fourth chemical functional group may be (tetrazine, cyclooctene), (alkyne group, azide group), (alkyne group, thiol group), (epoxy group, amine group), (epoxy group, thiol group), (acroyl group, amine group), (acroyl group, thiol group), (NHS ester group, amine group), (maleimide group, thiol group), (vinyl sulfone group, thiol group).

The first polymer and the first protein as described above have different chemical functional groups such that they can chemically bond by selective bonding between the different chemical functional groups. As a result, the first polymer and the first protein are coupled by a covalent interaction (or bond).

Similarly, the second polymer and the second protein as described above have different chemical functional groups such that they can chemically bond by selective bonding between the different chemical functional groups. As a result, the second polymer and the second protein are coupled by a covalent interaction (or bond).

In addition to the above described systems, the first or second polymer may be coupled to the first or second protein, respectively, using the the SpyTag/SpyCatcher system (see Zakeri, B., Fierer, J. O., Celik, E., Chittock, E. C., Schwarz-Linek, U., Moy, V. T. & Howarth, M. (2012). Peptide tag forming a rapid covalent bond to a protein, through engineering a bacterial adhesin. Proc. Natl. Acad. Sci. U.S.A. 109, E690-E697). The peptide SpyTag (13 amino acids) spontaneously reacts with the protein SpyCatcher (12.3 kDa) to form an intermolecular isopeptide bond between the pair. Hence, the SpyTag may be introduced into the first or second polymer and the SpyCatcher may be introduced into the first or second protein (or the other way round), leading to a covalent interaction between first or second polymer and the first or second protein, respectively, upon reaction of the SpyTag with the SpyCatcher. Alternative systems to the SpyTag/SpyCatcher are SnoopTag/SnoopCatcher, SnoopTagJr/SnoopCatcher, DogTag/SnoopTagJr.

Alternatively, an intein may be used to couple the first or second polymer to the first or second protein, respectively, via at least one covalent interaction. Inteins are intervening protein elements that can ligate flanking peptides (N and C-exteins) while excising themselves in a process called protein splicing. Since their discovery in 1990, more than 1500 inteins have been identified according to recent database analysis. The canonical protein splicing pathway involves a N-S or N-O acyl shift at the N-terminal splice site, the formation of a branched intermediate, cyclization of a conserved asparagine residue at the C-terminus of the intein to form a succinimide ring, and a final acyl shift to restore the peptide bond linking the ligated exteins. The result is excision of the intein and ligation of the flanking exteins. The amino acids strictly required for this single-turnover process are a cysteine or serine at the first position of the intein, referred to as the 1 position, and a cysteine, serine or threonine immediately downstream of the intein, referred to as the +1 position. In the present invention, a N-extein with a N-intein and a C-intein with a C-extein is fused to either the polymer or the protein, respectively. Complementation of the split intein will splice out the intein and covalently link the N-extein to the C-extein thus coupling the protein to the polymer.

When a first polymer or a second polymer is coupled to more than one first or second protein molecules, each protein molecule is coupled to the respective polymer as described above. Preferably, when a first polymer or a second polymer is coupled to more than one first or second protein molecules, the covalent interactions between the first polymer or the second polymer and the more than one first or second protein molecules, respectively, are the same.

In one embodiment, the first or second polymer can be coupled to the first or second protein, respectively, via at least one non-covalent interaction using the Streptavidin/Biotin system. Streptavidin is a 52.8 kDa protein which has an extraordinarily high affinity for biotin (also known as vitamin B7). With a dissociation constant (K_{d}) on the order of ≈10⁻¹⁴ mol/L, the binding of biotin to streptavidin is one of the strongest non-covalent interactions known in nature. Hence, Streptavidin may be introduced into the first or second polymer and biotin may be introduced into the first or second protein (or the other way round), leading to at least one non-covalent interaction between first or second polymer and the first or second protein, respectively, as soon as streptavidin interacts with biotin.

In another embodiment, the first or second polymer can be coupled to the first or second protein, respectively, via at least one non-covalent interaction using the His-tag/Ni-NTA system. The His-tag Ni-NTA interaction is based on the selectivity and high affinity of Ni-NTA (nickelnitrilotriacetic acid) for proteins containing an affinity tag of six or more consecutive Histidine residues. NTA, which has four chelating sites for nickel ions, is able to bind nickel tightly. Hence, Ni-NTA may be introduced into the first or second polymer and a His-tag may be introduced into the first or second protein (or the other way round), leading to at least one non-covalent interaction between first or second polymer and the first or second protein, respectively, as soon as Ni-NTA interacts with the His-tag.

Depending on the structure of the first and second polymers, more than one first proteins and second proteins can be coupled to the first polymer and the second polymer, respectively. For example, an 8-arm PEG molecule can be coupled to up to eight first protein molecules. In a preferred embodiment, the first proteins attached to the first polymer are identical. Accordingly, also the second proteins attached to the first second polymer are preferably identical.

The first polymer and the second polymer which are coupled to the first and second protein, respectively, may be different or the same. In a preferred embodiment, the first polymer and the second polymer are the same. According to the present invention, two polymers are the same if the only difference lies in the coupling region to the first or second protein, but otherwise the backbone of the polymers is identical.

The first polymer and the second polymer may be linear or multi-armed (branched). In order to facilitate cross-linking between the first and second polymers and, thus, formation of a 3D structure, the first polymer and the second polymer are preferably multi-armed.

The first polymer and the second polymer are selected from the group comprising a polyethylene glycol, a polyacrylamide, a polyacrylate, a poloxamer, a polysaccharide, DNA, polyvinyl alcohol, polylysine, polycaprolactone, a protein polymer and a copolymer thereof. Preferably, the first polymer and the second polymer are selected from the group comprising a polyethylene glycol, a polyacrylamide, a polyacrylate, a poloxamer, a polysaccharide, polyvinyl alcohol, polycaprolactone, a protein polymer and a copolymer thereof. More preferably, the first polymer and the second polymer are selected from the group comprising a polyethylene glycol, a polyacrylamide, a polyacrylate, a poloxamer, a polysaccharide, a protein polymer and a copolymer thereof. Most preferably, the first polymer and the second polymer are a polyethylene glycol.

Polyethylene glycol (PEG) is a synthetic polymer synthesized by ethylene oxide polymerization, which can be prepared with different chain lengths as well as different structures, such as linear or multi-armed structures. It is a favourable synthetic material because of its tailorable but typically strong mechanical properties that facilitate the bioprinting processes and shape maintenance of the deposited constructs.

In on embodiment, the polyethylene glycol is linear or multi-armed, preferably multi-armed, in order to facilitate cross-linking.

In one embodiment, a multi-arm polyethylene glycol includes a 3-arm, 4-arm, 6-arm and 8-arm polyethylene glycol, preferably a 6-arm and 8-arm polyethylene glycol, more preferably an 8-arm polyethylene glycol.

In one embodiment, the polyethylene glycol may have an average molecular weight of 0.5 kDa to 1000 kDa, preferably 1 kDa to 250 kDa, more preferably, 5 kDa to 100 kDa, most preferably 10 kDa to 75 kDa, as determined by gel permeation chromatography (GPC).

Polyacrylamide (poly(2-propenamide)) is a polymer (-CH₂CHCONH₂-) formed from acrylamide subunits. In one embodiment, it has a linear-chain structure, a star structure or is branched. Preferably, the polyacrylamide has a star structure or is branched.

In one embodiment, the polyacrylamide may have an average molecular weight of 0.5 kDa to 1000 kDa, preferably 1 kDa to 250 kDa, more preferably, 5 kDa to 100 kDa, as determined by gel permeation chromatography (GPC).

A polyacrylate is a polymer of acrylic monomers. In one embodiment, acrylic monomers are selected from the group comprising methacrylates, methyl acrylate, ethyl acrylate, 2-chloroethyl vinyl ether, 2-ethylhexyl acrylate, hydroxyethyl methacrylate, butyl acrylate, butyl methacrylate, trimethylolpropane triacrylate (TMPTA) or mixtures thereof. Methacrylates are derivatives of methacrylic acid and include the parent acid (CH₂C(CH₃)CO₂H), salts (e.g., CH₂C(CH₃)CO⁻2Na⁺), esters (e.g. methyl methacrylate). A preferred polyacrylate is poly(2-hydroxyethyl methacrylate) (pHEMA). In one embodiment, the polyacrylate may be linear or branched, preferably the polyacrylate is branched.

In one embodiment, the polyacrylate may have an average molecular weight of 0.5 kDa to 1000 kDa, preferably 1 kDa to 250 kDa, more preferably, 5 kDa to 100 kDa, as determined by gel permeation chromatography (GPC).

Poloxamers are nonionic triblock copolymers composed of a central hydrophobic chain of polyoxypropylene (poly(propylene oxide)) flanked by two hydrophilic chains of polyoxyethylene (poly(ethylene oxide)). They are also known by the trade names Synperonics®, Pluronic®, and Kolliphor®. In one embodiment, the poloxamer may have an average molecular weight of 0.1 kDa to 1000 kDa, preferably 0.5 kDa to 250 kDa, more preferably, 3 kDa to 100 kDa, as determined by gel permeation chromatography (GPC). In one embodiment, the content of polyoxyethylene in the poloxamer is from 30% to 90%, preferably from 40% to 80%, based on the weight of the poloxamer.

A polysaccharide is a long chain of carbohydrate molecules, specifically a polymeric carbohydrate composed of monosaccharide units bound together by glycosidic linkages. In one embodiment of the invention, the polysaccharide is selected from the group consisting of hyaluronic acid, chitosan, agarose, alginic acid or alginates.

Hyaluronic acid is an anionic, nonsulfated glycosaminoglycan distributed widely throughout connective, epithelial, and neural tissues. Preferably, the average molecular weight of hyaluronic acid is 10 kDa to 4000 kDa, more preferably 50 kDa to 2000 kDa, as determined by gel permeation chromatography (GPC).

Chitosan is a linear polysaccharide composed of randomly distributed β-(1→4)-linked D-glucosamine (deacetylated unit) and N-acetyl-D-glucosamine (acetylated unit). It is made by treating the chitin shells of shrimp and other crustaceans with an alkaline substance, like sodium hydroxide. Preferably, the average molecular weight of chitosan is 10 kDa to 500 kDa, more preferably 40 kDa to 350 kDa, as determined by gel permeation chromatography (GPC).

Agarose is a linear polymer and consists of alternating D-galactose and 3,6-anhydro-L-galactopyranose linked by α-(1→3) and β-(1→4) glycosidic bonds. The 3,6-anhydro-L-galactopyranose is an L-galactose with an anhydro bridge between the 3 and 6 positions, although some L-galactose units in the polymer may not contain the bridge. Some D-galactose and L-galactose units can be methylated, and pyruvate and sulfate may also be found in small quantities. Preferably, the average molecular weight of agarose is 10 kDa to 500 kDa, more preferably 50 kDa to 250 kDa, as determined by gel permeation chromatography (GPC).

Alginic acid, also called algin, is a polysaccharide distributed widely in the cell walls of brown algae that is hydrophilic. With metals such as sodium and calcium, its salts are known as alginates. Alginic acid is a linear copolymer with homopolymeric blocks of (1-4)-linked β-D-mannuronate (M) and its C-5 epimer α-L-guluronate (G) residues, respectively, covalently linked together in different sequences or blocks. The monomers may appear in homopolymeric blocks of consecutive G-residues (G-blocks), consecutive M-residues (M-blocks) or alternating M and G-residues (MG-blocks). Preferably, the average molecular weight of alginic acid or alginate is 1 kDa to 1000 kDa, more preferably 5 kDa to 750 kDa, even more preferably 10 kDa to 500 kDa, as determined by gel permeation chromatography (GPC).

Polyvinyl alcohol as used herein may have an average molecular weight of 10 kDa to 500 kDa, more preferably 50 kDa to 250 kDa, as determined by gel permeation chromatography (GPC).

Polylysine refers to several types of lysine homopolymers, which may differ from each other in terms of stereochemistry and link position. In one embodiment, polylysine is α-polylysine or ε-polylysine, preferably ε-polylysine. α-Polylysine is a synthetic polymer, which can be composed of either L-lysine or D-lysine. This results in poly-L-lysine (PLL) and poly-D-lysine (PDL), respectively. ε-Polylysine is preferagly ε-poly-L-lysine (EPL). Polylysine preferably contains 10-50, more preferably 20 to 35 L-lysine residues.

Polycaprolactone or (1,7)-polyoxepan-2-one may have an average molecular weight of 0.5 kDa to 1000 kDa, preferably 1 kDa to 250 kDa, more preferably, 5 kDa to 100 kDa, as determined by gel permeation chromatography (GPC).

Protein polymers suitable for the present invention comprise collagen, fibrin/fibrinogen and gelatin as well as proteins based on GB1, elastin and resilin.

As copolymer, a linear or branched copolymer can be used, preferred is a branched copolymer. The copolymer can be an alternating copolymer, a random copolymer, a block copolymer or a graft copolymer. In an embodiment, the copolymer contains two, three or four different types of monomers or polymers, preferably two or three different types of monomers or polymers, more preferably two different types of monomers or polymers. In an embodiment, the copolymer is a copolymer of polyethylene glycol with a polyacrylamide, a polyacrylate, a poloxamer, a polysaccharide, polyvinyl alcohol, polylysine or polycaprolactone. In another embodiment, the copolymer is a copolymer of a polyacrylamide with a polyacrylate, a poloxamer, a polysaccharide, polyvinyl alcohol, polylysine or polycaprolactone. In another embodiment, the copolymer is a copolymer of a polyacrylate with a poloxamer, a polysaccharide, polyvinyl alcohol, polylysine or polycaprolactone. In another embodiment, the copolymer is a copolymer of a polysaccharide with polyvinyl alcohol, polylysine or polycaprolactone.

In a further embodiment of the invention, the bioink contains at least one cell adhesion motif. The cell adhesion motif may facilitate cell adhesion and cell spreading once a hydrogel is formed using the bioink of the present invention. In one embodiment, the first polymer coupled to the first protein and/or the second polymer coupled to the second protein comprise at least one cell adhesion motif. That is, the at least one cell adhesion motif is contained in the first/second polymer or in the first/second protein. In a further embodiment, the at least one cell adhesion motif is contained in the first protein and/or the second protein. When more than one cell adhesion motif is contained in the bioink, the cell adhesion motifs can be the same (e.g. a tandem repeat of the same cell adhesion motif) or different. A cell adhesion motif is preferably contained in the protein of the present invention, if the first and/or second polymer do not induce cell adhesion, for example if the polymer is PEG.

In one embodiment, the at least one cell adhesion motif is selected from the group consisting of RGD, GFOGER, YGISR and A5G81. The at least one cell adhesion motif is preferably RGD. RGD stands for the tripeptide Arg-Gly-Asp. GFOGER stands for Gly-Phe-hydroxyPro-Gly-Glu-Arg and is preferably in triple-helical conformation. YGISR stands for Tyr-Gly-Ile-Ser-Arg. A5G81 is a peptide having the sequence AGQWHRVSVRWGC which stands for Ala-Gly-Gln-Trp-His-Arg-Val-Ser-Val-Arg-Trp-Gly-Cys.

In a further embodiment, the concentration of the first and second proteins coupled to the first and second polymers, respectively, in the bioink may be 5 to 500 mg/mL, preferably 10 to 100 mg/mL, as determined by using known protein assay methods, preferably by the Bradford assay. Since higher concentrations of proteins in the bioink lead to hydrogels having increased stiffness, the concentration of proteins in the bioink can be varied depending on the desired properties of the hydrogel.

In a further embodiment, the mole ratio of first protein to second protein in the bioink is preferably 8:1 to 1:8, more preferably 3:1 to 1:3, even more preferably 1.5:1 to 1:1.5, most preferably 1:1. When the mole ratio of first protein to second protein is 1:1, all the first proteins can form a heterodimer with the second proteins.

The bioink of the present invention is liquid, i.e. it has a viscosity that allows the bioink to be printed using common methods, as described below.

In a further embodiment, the bioink may further include a solvent such distilled water or a buffer solution. Specifically, the buffer solution includes, but is not limited to, one or more selected from the group consisting of 2-(n-morpholino)ethanesulfonic acid (MES), Tris-buffered saline (TBS), 4-(4,6-dimethoxy-1,3,5-tiazin-2-yl)-4-ethylmorpholinium chloride (DMTMM) and phosphate buffer saline.

Additionally, the bioink may further include one or more selected from the group consisting of cells, a dye, a drug, a viscosity enhancer, a regulator material of differentiation, a cell culture medium and a substance that inhibits cell death.

Specifically, the cells may preferably but without limitation be one or more selected from the group consisting of human-derived stem cells, muscle-derived stem cells, dental pulp stem cells, nasal concha-derived mesenchymal stromal cells, an osteoblast, a myoblast, a tenocyte, a neuroblast, a fibroblast, a glioblast, a germ cell, hepatocyte, a renal cell, a sertoli cell, a chondrocyte, an epithelial cell, a cardiovascular cell, a keratinocyte, a smooth muscle cell, a cardiomyocyte, glial cell, a endothelial cell, a hormone-secreting cell, an immune cell, a pancreatic islet cell and a neuron.

Also, as the dye, fluorescein isothiocyanate (FITC), rhodamine, IR 780, IR 783, or propidium iodide (PI) may be used.

As the drug, a generally used antibiotic, anticancer agent, inflammatory analgesic agent, antiviral agent, antibacterial agent, protein or peptide may be used.

As viscosity enhancer, the viscosity enhancer may be selected from ones suitable for maintaining excellent printing properties and the initial strength of the bio-ink. Examples include hyaluronic acid and dextran.

The regulator material of differentiation may be included to induce the differentiation of stem cells (including embryonic stem cells, inducible pluripotent stem cells, and adult stem cells) contained in the bioink into specific cells. Examples include chemicals, growth factors such as bone morphogenetic protein-2 (BMP-2) when inducing differentiation of bio-printed stem cells into bone cells, TGF-beta3 when inducing differentiation into chondrocytes, dexamethasone when inducing differentiation into osteocytes, and 5-azacytidine (5-azacytidine) when inducing differentiation into muscle cells. In addition, the bioink of the present invention may further comprise cytokines, peptides, or low-molecular chemicals. In this way, the bioink of the present invention may comprise growth factors, cytokines, peptides, low-molecular chemicals, or the like capable of inducing differentiation of stem cells into specific tissue cells. Thus, it can be utilized as a functional bioink capable of inducing the tissue-like structure including the prepared stem cells to be differentiated into a specific tissue after printing.

The cell culture medium is meant to include any medium suitable for a desired cell. Examples of cell culture medium include Dulbecco's phosphate buffered saline, Earle's balanced salts, Hank's balanced salts, Tyrode's salts, Alsever's solution, Gey's balanced salt solution, Kreb's-Henseleit buffer modified, Kreb's-ringer Bicarbonate Buffer, Puck's saline, Dulbecco's Modified Eagle's medium (DMEM), Dulbecco's Modified Eagle's medium (DMEM)/ Nutrient Mixture F-12 Ham, nutrient mixture F-10 ham (Ham's F-10), medium 199, Eagle's minimal essential medium (EMEM), RPMI 1640 Medium, Ames' Media, BGJb medium (Fitton-Jackson modification), Click's medium, CMRL-1066 medium, Fischer's medium, Glascow Minimum Essential Medium (GMEM), Iscove's Modified Dulbecco's Medium (IMDM), L-15 medium (Leibovitz), McCoy's 5A Modified medium, NCTC medium, Swim's S-77 medium, Waymouth medium, William's E Medium, or combinations thereof, but are not limited thereto. In addition, the cell culture medium may further contain serum (e.g. fetal calf serum), antibiotics such as penicillin or streptomycin, albumin, selenium, transferrin, fetuin, sugars, amino acids, vitamins, growth factors, cytokines, hormones, lipids, lipid carriers, and cyclodextrins or combinations thereof.

Examples of a substance that inhibits cell death may be selected from small molecules, antibodies, peptides, peptibodies, anti-TNF substances, substances inhibiting the activity of interleukins, substances inhibiting the activity of interferons, substances inhibiting the activity of GCSF (granulocyte colony-stimulating factor), substances inhibiting the activity of macrophage inflammatory protein, substances inhibiting the activity of TGF-B (transformation growth factor B), substances inhibiting the activity of MMP (matrix metalloproteinase), substances inhibiting the activity of the MAPK/JNK signaling cascade, substances inhibiting the activity of Src kinase, a substance inhibiting the activity of JAK (Janus kinase), or combinations thereof.

### Cartridge for 3D printer

The present invention may provide a cartridge for a 3D printer or a 3D printer which contains the bioink. The detailed description of the bioink has been provided above.

### Method for preparing a hydrogel and a hydrogel

The present invention provides a method for reversibly preparing a hydrogel, including:
(i) preparing a bioink comprising a first polymer coupled to a first protein and a second polymer coupled to a second protein, wherein the first protein and the second protein reversibly form a heterodimer upon irradiation with visible or infrared light in a specific wavelength range;
(ii) printing the bioink, wherein printing comprises irradiating the bioink with visible or infrared light in a first wavelength range to obtain a hydrogel.

The detailed descriptions of the first polymer, the second polymer, the first protein and the second protein as well as of the coupling between the first polymer or the second polymer and the first protein or the second protein, respectively, have been provided above.

Step (ii) of printing the bioink can be carried out by stereolithography, laser-assisted printing, inkjet printing, multi-jet modelling, extrusion printing, and granule-based medium-assisted printing. Preferably, printing of the bioink is carried out by stereolithography, laser-assisted printing or extrusion printing. More preferably, printing of the bioink is carried out by extrusion printing.

Stereolithography printing is based on polymerization of light-sensitive polymers by precisely controlled light glinted from digital micromirrors. In comparison with other methods, stereolithography is a technique with high printing quality, speed, and cell viability. In one embodiment, stereolithography printing may be implemented using direct-laser writing, physical mask projection, or digital mask projection machines.

Laser-assisted printing offers the direct deposition of materials on a free surface based on the "aim-and-shoot" procedure. A laser-assisted printing system typically consists of a laser-absorbing layer, called the ribbon, a feeding layer of cell-laden hydrogel beneath, and a receiving substrate. When the laser pulse is focused on the laser-absorbing layer (the "aim" step), a vapor pocket is generated in the feeding layer, resulting in the falling off of the cell-laden droplet (the "shoot" step) to the receiving substrate. This technology offers a high owing to the accuracy of laser targeting itself.

In inkjet printing, an inkjet cartridge typically generates cell-laden drops and ejects them onto a substrate, called bio-paper, in a "drop-on-demand" fashion. The drops can be separated from a continuous flow of bioink passing through the nozzle by generating a bubble in the flow. The bubble is usually produced by local heating of the nozzle or by physical breaking using a piezoelectric actuator. The heating method instantaneously increases the temperature to evaporate the ink, making the bubble and the ink droplet. On the other hand, a piezoelectric actuator physically breaks the ink and is therefore preferred for application in cell printing.

To construct a 3D shape by stacking the ejected droplets, the multi-jet modelling method was developed by incorporating photo-polymerization into inkjet printing systems. The configuration is generally composed of inkjet heads, a light irradiator, and a building platform with an elevator. The inkjet heads deliver photopolymers onto the tray, and the lamp is moved directly above the ejected droplet for rapid polymerization.

Extrusion-based printing systems typically move a syringe containing the materials in 3D space and dispense a continuous stream by piston-driven or pneumatic forces. Hence, this method enables printing higher concentrations of bioinks (generally having viscosity in the range of 30 - 6 × 10⁷ mPa s) and brings unique benefits when printing large volume cell-laden constructs. Inkjet-based printing can jet 1-10000 droplets/s, but the thickness of the printed strut is also limited due to the low concentration of hydrogel. In this sense, extrusion-based printing is advantageous for the fast generation of large constructs. Moreover, this printing method offers not only various options for selecting building materials, including thermoplastic polymers, hydrogels, and cells, but also combinatorial construction by simply alternating the printing heads containing different materials. Common extrusion-based printing methods are categorized into pneumatic, piston-driven, and screw-driven dispensing. In pneumatic dispensing, air pressure provides the required driving force, while in piston and screw-driven dispensing, vertical and rotational mechanical forces initiate printing, respectively.

Granule-based medium-assisted printing involves a gelatin slurry support bath to maintain the shape of the printed precursor during its complete gelation. After removing the liquid support, a complex biomimetic structure can be obtained.

In order to form a hydrogel, the printing step comprises irradiating the bioink with visible or infrared light in a first wavelength range. In an embodiment, the irradiation step may be included in the printing step or may be carried out following the printing step. For example, hydrogel formation by irradiating the bioink with visible or infrared light in a first wavelength range may be followed printing of the bioink by extrusion.

By the irradiation, the first protein and the second protein form a heterodimer, thus cross-linking the first polymer and the second polymer of the bioink, to which the first protein and second protein are coupled. This light-triggered cross-linking leads to gelation of the bioink and thus formation of a hydrogel. Advantageously, in the present invention hydrogel formation is not triggered by UV light, and it thus has minimal impact on cells that may be contained in the bioink.

The wavelength range of the light used to irradiate the bioink during or after the printing step depends on the first protein in the bioink. Specifically, the wavelength range is in the visible light range (400 to less than 700 nm) or in the infrared range (700-1000 nm), in particular in the near-infrared range (700-790 nm).

When the first protein is a phytochrome and the second protein is a phytochrome interacting partner, heterodimerization occurs by irradiating the phytochrome and the phytochrome interacting partner with visible or infrared light in a first specific wavelength range, wherein this range preferably includes the wavelength of maximum absorbance of the phytochrome. The heterodimerization can then be reversed by irradiating the heterodimer with visible or infrared light in a second specific wavelength range, wherein this range preferably includes the wavelength of maximum absorbance of the phytochrome in the heterodimer.

Specifically, when the phytochrome in the ground state is irradiated with a wavelength corresponding to the wavelength of maximum absorbance of the phytochrome in the ground state, the highest activation of the phytochrome, i.e. the highest concentration of phytochrome in the activated state, is observed.

Accordingly, when the heterodimer, i.e. the phytochrome in the activated state, is irradiated with a wavelength corresponding to the wavelength of maximum absorbance of the phytochrome in the activated state, the highest concentration of phytochrome in the ground state is obtained, specifically all phytochrome molecules are in the ground state.

When the phytochrome in the ground state is irradiated with light having a wavelength between the wavelength of maximum absorbance of the phytochrome in the ground state and the wavelength of maximum absorbance of the phytochrome in the activated state, the concentration of phytochrome in the activated state will be reduced compared to irradiation of the phytochrome in the ground state with light having the wavelength of maximum absorbance of the phytochrome in the ground state. The degree of reduction of activation increases with increasing distance from the wavelength of maximum absorbance of the phytochrome in the ground state. The same applies for the phytochrome in the activated state. When the phytochrome in the activated state (i.e. in the heterodimer) is irradiated with light having a wavelength between the wavelength of maximum absorbance of the phytochrome in the ground state and the wavelength of maximum absorbance of the phytochrome in the activated state, the concentration of phytochrome in the ground state will be reduced compared to irradiation of the phytochrome in the activated state with light having the wavelength of maximum absorbance of the phytochrome in the activated state. The degree of reduction of inactivation increases with increasing distance from the wavelength of maximum absorbance of the phytochrome in the activated state. Hence, between the wavelength of maximum absorbance of the phytochrome in the ground state and the wavelength of maximum absorbance of the phytochrome in the activated state, a mixture of phytochrome in the ground state and in the activated state in varying ratios is present. This corresponds to a different concentration of cross-links in the hydrogel of the present invention.

In one embodiment, the first wavelength range includes the absorbance maximum of the ground state which converts the ground state of the phytochrome to its activated state, but it does not include the wavelength of absorbance maximum of the activated state which converts the activated state of the phytochrome to its ground state. Preferably, the wavelength of the absorbance maximum of the ground state of the phytochrome has the highest intensity in the first wavelength range. Accordingly, the second wavelength range includes the wavelength of absorbance maximum of the activated state which converts the activated state of the phytochrome to its ground state, but it does not include the wavelength of absorbance maximum of the ground state which converts the ground state of the phytochrome to its activated state. Preferably, the wavelength of the absorbance maximum of the activated state of the phytochrome has the highest intensity in the second wavelength range.

In another embodiment, which is less preferred, the first wavelength range includes the wavelength of absorbance maximum of the ground state which converts the ground state of the phytochrome to its activated state, and it also includes the wavelength of absorbance maximum of the activated state which converts the activated state of the phytochrome to its ground state. However, in the first wavelength range the intensity of the wavelength of the absorbance maximum of the ground state is higher than the intensity of the wavelength of the absorbance maximum of the activated state. Accordingly, the second wavelength range includes the wavelength of absorbance maximum of the activated state which converts the activated state of the phytochrome to its ground state, and it also includes the wavelength of absorbance maximum of the ground state which converts the ground state of the phytochrome to its activated state. However, in the second wavelength range the intensity of the wavelength of the absorbance maximum of the activated state is higher than the intensity of the wavelength of the absorbance maximum of the ground state. Hence, in an embodiment of the invention, the first and second wavelength ranges can be identical from the point of view of the wavelengths, but are different from the point of view of the intensity of each wavelength.

In an embodiment, the wavelengths of the absorbance maximum of the ground state or the activated state do not need to be the peak wavelengths in the first or second wavelength range. In cases where the wavelengths of the absorbance maximum of the ground state or the activated state are not the peak wavelengths in the first or second wavelength range, higher light intensities may be needed for hydrogel formation or hydrogel dissolution. However, if the wavelengths of the absorbance maximum of the ground state or the activated state are the peak wavelengths in the first or second wavelength range, a minimum of light intensity is needed for hydrogel formation or hydrogel dissolution. Therefore, it is preferred that the wavelength of the absorbance maximum of the ground state in step ii) and the wavelength of the absorbance maximum of the activated state in step iii) described below are the peak wavelengths in the first and second wavelength range.

As mentioned above, in case the first protein is a phytochrome comprising PhyA, PhyB, PhyC, PhyD, PhyE, preferably comprising PhyA or PhyB, the first wavelength range preferably has the wavelength of 660 nm as peak wavelength. Preferably, heterodimerization occurs by irradiating the bioink with light in a first wavelength range of 400 to less than 700 nm, more preferably 600-690 nm, even more preferably from 640-680 nm, most preferably 650-670 nm.

When the phytochrome is BphP1, the first wavelength range preferably has the wavelength of 760 nm as peak wavelength.

In case the first protein is a phytochrome comprising BphP1, heterodimerization occurs by irradiating the bioink with light preferably in a wavelength range of 700-900 nm, more preferably 720-780 nm, even more preferably from 750-770 nm.

As mentioned above, the second protein is preferably a phytochrome interacting partner which is selected in an embodiment from the group consisting of PIF1, PIF2, PIF3, PIF4, PIF5, PIF6, PIF7, PIF8, FHY1, FHL, PpsR2, Q-PAS1. The second protein is preferably selected from the group consisting of PIF3, PIF6, FHY1, FHL, PpsR2 and Q-PAS1.

Both PhyA and PhyB form heterodimers with PIF3, PIF6, FHY1, FHL, leading to heterodimers PhyA/PIF3, PhyA/PIF6, PhyA/FHY1, PhyA/FHL, PhyB/PIF3, PhyB/PIF6, PhyB/FHY1, PhyB/FHL.

BphP1 forms heterodimers with both PpsR2 and Q-PAS1, leading to heterodimers BphP1/PpsR2 and BphP1/Q-PAS1.

In a preferred embodiment, the first protein and the second protein form heterodimers PhyA/PIF3, PhyA/PIF6, PhyA/FHY1, PhyA/FHL, PhyB/PIF3, PhyB/PIF6, PhyB/FHY1, PhyB/FHL, BphP1/PpsR2 and BphP1/Q-PAS1. More preferably, the heterodimers are PhyA/FHY1, PhyA/FHL, PhyB/PIF3, PhyB/PIF6, BphP1/PpsR2 and BphP1/Q-PAS1. Most preferably, the heterodimers are PhyB/PIF3 and PhyB/PIF6.

Accordingly, PhyA/PIF3, PhyA/PIF6, PhyA/FHY1, PhyA/FHL, PhyB/PIF3, PhyB/PIF6, PhyB/FHY1 and PhyB/FHL heterodimers are formed by irradiating the corresponding monomers with light preferably in a first wavelength range having the wavelength of 660 nm as peak wavelength. Preferably, the first wavelength range is of 400 to less than 700 nm, more preferably 600-690 nm, even more preferably from 640-680 nm, most preferably 650-670 nm.

Further, BphP1/PpsR2 and BphP1/Q-PAS1 heterodimers are formed by irradiating the corresponding monomers with light preferably in a first wavelength range having the wavelength of 760 nm as peak wavelength. Preferably, the wavelength range is of 700-900 nm, more preferably 720-780 nm, even more preferably from 750-770 nm.

In an embodiment, irradiating the bioink is carried out with a suitable light source, such as a LED, that emits light preferably of a specific wavelength or in a narrow wavelength range (max. 80 nm). In an embodiment, irradiation is carried out using a confocal microscope equipped with a red (e.g. 660 nm) and/or far-red (e.g. 740 nm) laser.

In an embodiment, the distance from the light source to the printed bioink is preferably 1 cm to 30 cm.

In an embodiment, the photon intensity of the light is preferably 1-1000 µmol m⁻² s⁻¹, more preferably 50 to 500 µmol m⁻² s⁻¹.

In a further embodiment, the bioink is irradiated with light in a first wavelength range for a time period of 1 µs to 10 min, preferably 1 ms to 1 min, more preferably 100 ms to 10 s. At the end of this irradiation period, photoswitching is preferably completed. Complete crosslinking within the bioink by heterodimerization can be delayed in the range of seconds. Completion of crosslinking can be detected by measuring the mechanical and rheological properties of the gel (as discussed below).

In the method of the present invention, heterodimerization of the first and second protein is preferably dependent on the dose of light. This means that the higher the light intensity at a given first wavelength range, the quicker is the heterodimerization, and therefore the formation of the hydrogel.

The mechanical and rheological properties of the hydrogel can be controlled by changing the concentration of the first and second proteins in the bioink. In an embodiment, the concentration of the first and second proteins coupled to the first and second polymers in the bioink may be 5 to 500 mg/mL, preferably 10 to 100 mg/mL, as determined by methods described above.

Additionally, also the type of first and second polymers may have an influence on the properties of the hydrogel. Within the same category of polymers, polymers which are coupled to a higher number of first and second proteins will be able to form a higher number of cross-links which will have an impact on the mechanical properties of the hydrogels obtained therefrom.

Optionally, before irradiating the bioink with visible or infrared light in a first wavelength range, thus forming a hydrogel, the bioink may be irradiated with visible or infrared light in a second wavelength range, in order to prevent undesired hydrogel formation during step ii). Using the above example in which hydrogel formation is followed printing of the bioink by extrusion, since a hydrogel formation is undesired during extrusion, the bioink which is printed may be irradiated with visible or infrared light in a second wavelength range, under which conditions heterodimerization of the first protein and second protein is suppressed. The details of the visible or infrared light in a second wavelength range are given below in step iii).

In a further embodiment of the method of the present invention, the method further comprises the step of
(iii) irradiating the hydrogel with visible or infrared light in a second wavelength range to dissolve the hydrogel, wherein the second wavelength range of the light in step (iii) is different from the first wavelength range of the light in step (ii).

The second wavelength range is in the visible light range (400 to less than 700 nm) or in the infrared range (700-1000 nm), in particular in the near-infrared range (700-790 nm). According to an embodiment, if the first wavelength range is in the visible light range (400 to less than 700 nm), the second wavelength range is preferably in the infrared range (700-1000 nm), in particular in the near-infrared range (700-790 nm). Accordingly, if the first wavelength range is in the infrared range (700-1000 nm), in particular in the near-infrared range (700-790 nm), the second wavelength range is preferably in the visible light range (400 to less than 700 nm).

Heterodimerization which was triggered in step (ii) by irradiating the bioink with visible or infrared light in a first wavelength range can thus be reversed by irradiating the hydrogel with visible or infrared light in a second wavelength range, wherein the first and second wavelength ranges are different. This has been described above.

In an embodiment of the present invention, if the first wavelength range in step (ii) was in the visible light range (400to less than 700 nm), the second wavelength range in step (iii) is preferably in the infrared range (700-1000 nm), more preferably in the near-infrared range (700-790 nm). Accordingly, if the first wavelength range in step (ii) was in the infrared range (700-1000 nm), preferably in the near-infrared range (700-790 nm), the second wavelength range in step (iii) is preferably in the visible light range (400 to less than 700 nm).

Reversion of the heterodimerization occurs in case of PhyA to PhyE by irradiating the heterodimer with light in a second wavelength range preferably having the wavelength of 740 nm as peak wavelength. Further, the second wavelength range is preferably of 700-790 nm, more preferably 720-770 nm, even more preferably from 730-750 nm.

When the phytochrome is BphP1, the second wavelength range preferably has the wavelength of 640 nm as peak wavelength.

Reversion of the heterodimerization occurs in case of BphP1 by irradiating the heterodimer with light preferably in a wavelength range of 500-690 nm, more preferably 620-670 nm, even more preferably from 630-650 nm.

Accordingly, PhyA/PIF3, PhyA/PIF6, PhyA/FHY1, PhyA/FHL, PhyB/PIF3, PhyB/PIF6, PhyB/FHY1 and PhyB/FHL heterodimers are reversed by irradiating the heterodimers with light preferably in a second wavelength range having the wavelength of 740 nm as peak wavelength. Preferably, the second wavelength range is of 700-790 nm, more preferably 720-770 nm, even more preferably from 730-750 nm.

Further, BphP1/PpsR2 and BphP1/Q-PAS1 heterodimers are reversed by irradiating the heterodimers with light preferably in a second wavelength range having the wavelength of 640 nm as peak wavelength. Preferably, the second wavelength range is of 500 to less than 700 nm, more preferably 620-670 nm, even more preferably from 630-650 nm.

In an embodiment, irradiating the hydrogel is carried out with a suitable light source, such as a LED, that emits light preferably of a specific wavelength or in a narrow wavelength range (max. 80 nm). In an embodiment, irradiation is carried out using a confocal microscope equipped with a red (e.g. 660 nm) and/or far-red (e.g. 740 nm) laser.

In an embodiment, the distance from the light source to the hydrogel is preferably 1 cm to 30 cm.

In an embodiment, the photon intensity of the light (light flux) is preferably 1-1000 µmol m⁻² s⁻¹, more preferably 50 to 500 µmol m⁻² s⁻¹.

In a further embodiment, the hydrogel is irradiated with light in a second wavelength range for a time period of 1 µs to 10 min, preferably 1 ms to 1 min, more preferably 100 ms to 10 s.

Preferably, the second wavelength range is selected to lead to a complete dissolution of the hydrogel. A completely dissolved hydrogel has preferably the same mechanical and rheological properties of the bioink, which can be easily determined by a person skilled in the art.

In a further aspect of the invention, a hydrogel is provided, which is reversibly formed by irradiating the bioink with visible or infrared light in a specific wavelength range. The detailed description of the bioink has been provided above. Also the detailed description of the method for reversibly forming the hydrogel has been provided above.

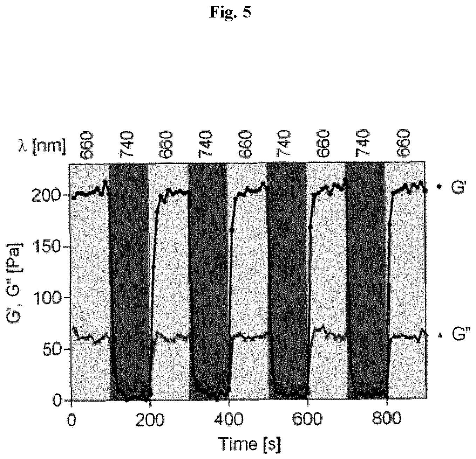

In one embodiment, the bioink of the present invention can be cross-linked and then dissolved in a reversible manner for at least 5 times (as shown in Fig. 5) and up to 100 times without substantial change in the mechanical and rheological properties of the bioink or hydrogel. The storage modulus G' and loss module G" may each differ in each cross-linking/dissolution cycle 0-10%, preferably 0-5% from the preceding cross-linking/dissolution cycle.

In an embodiment, the hydrogel has a Young's modulus of 100 Pa to 500 kPa, preferably 0.5 kPa to 100 kPa, as a parameter describing the stiffness of the hydrogel.

As explained above, the hydrogel of the present invention can be formed by irradiating the bioink of the present invention with visible or infrared light in a first wavelength range. The so formed hydrogel can then be dissolved by irradiating the bioink of the present invention with visible or infrared light in a second wavelength range. Changes in the rheological properties caused by irradiation with light in a first or a second wavelength range can be determined as explained below using e.g. a rheometer with parallel plate configuration. The hydrogel of the present invention may have a desired storage modulus G' and a loss module G" when irradiated with light in a first wavelength range. Upon dissolution by irradiation with light in a second wavelength range, the bioink can be re-obtained which, as explained above, is liquid. Accordingly, the storage modulus G' and a loss module G" of the dissolved hydrogel are low, preferably lower than 25 Pa, as determined in the experimental section.

Similarly, also the Young's modulus of the hydrogel is higher than the dissolved hydrogel, as determined by indentation-type atomic force microscopy (AFM). Here, the Young's modulus is determined from Hertz-fits to force-distance curves acquired with a spherical nanoindenter.

The mechanical and rheological properties of the hydrogel of the present invention can be tuned by irradiating the hydrogel with light in a wavelength range between the first and the second wavelength ranges. Within this range, dimerization of the first and second proteins is not completely reversed, i.e. not all cross-links are broken, so that dissolution of the hydrogel will not be complete. Accordingly, the Young's modulus as well as the storage modulus G' will be reduced, but will be higher than for the completely dissolved hydrogel.

This will be explained using the combination phyB/PIF6 as an example. In a bioink comprising phyB as first protein and PIF6 as second protein, the most preferred first wavelength range includes 660 nm and the most preferred second wavelength range includes 740 nm. Hence, irradiation of the bioink with light in a wavelength range including 660 nm will lead to heterodimerization of phyB/PIF6 and, consequently, to the formation of a hydrogel (see Fig. 1). Irradiation of the hydrogel with light in a wavelength range including 740 nm will lead to the complete dissolution of the hydrogel. However, irradiating the hydrogel with light in a wavelength range between the first wavelength range including 660 nm and the second wavelength range including 740 nm will lead to a reduced stiffness of the hydrogel. Hence, within this range, the rheological and mechanical properties of the hydrogel can be tuned. In particular, gradually shifting the irradiation wavelength range from a range including 740 nm to a range including 660 nm leads to a gradual decrease of G', so that any intermediate stiffness can be set by choosing the appropriate irradiation wavelength range.

Accordingly, the mechanical and rheological properties of the hydrogel can be adjusted over time to optimally match the demands of the given biomedical application.

In an embodiment of the invention, the hydrogels of the present invention share the hallmarks of biological optogenetic systems, in which the output signal is i) fully reversible by alternating the irradiation input, ii) dose-dependent by adjusting the light flux, iii) adjustable by different irradiation wavelengths, and iv) subject to precise spatial control.

In a further aspect, desired tissue-like organs can be obtained by the printing process and by the light-triggered cross-linking and thus hydrogel forming process of the present invention. The tissue-like organ may be cultured in a culture medium. The culture and culture medium are as described above.

A tissue-like organ can thus be obtained using a hydrogel of the present invention. 3D bioprinting technology has the potential to prepare a tissue or organ structure clinically applicable in terms of clinical shape and size. Through the combination of tissue-specific cell types and bioinks applied to 3D structures, the reproductive ability inherent in the cell can be utilized, thereby leading to the possibility of preparing the desired tissue or organ. In the 3D printed tissue structure, the size of the nozzle allows the integration of cell-containing bioink to be finely manipulated in terms of volume and position. In addition, the geometric and constitutive structures of the tissue-like organ may be controlled. Accordingly, the printed tissue structures using the bioink according to the present invention provide structures that are anatomically and functionally similar to mammalian tissues and organs.

For example, by forming a tissue structure printed using hepatocytes and vascular cells, a tissue structure printed using osteocytes and chondrocytes, a tissue structure printed using muscle cells and tendon cells, and tissue structure printed using neurons and muscle cells, a structure similar in function to the actual tissue structure in the body may be produced.

Once the tissue-like organ is cultured and the cells have secreted their own extracellular matrix, the scaffold material (i.e. the hydrogel) can be dissolved with minimal impact on the cells by irradiating the hydrogel with visible or infrared light in a second wavelength range, as explained in detail in combination with step iii) of the method of the present invention.

In a further embodiment, the hydrogel of the present invention can be used in drug discovery and development processes. The hydrogel may be printed in a range of 3D culture systems and human tissue models to produce better *in vitro* testing by generating models with improved physiological relevance and high reproducibility. By applying bioprinting in drug discovery and development ineffective or harmful drugs can be identified earlier in the discovery process and resources can be shifted to more promising drug candidates. The cost of drug development caused by clinical trial failures can also be reduced.

In such a drug discovery or development process, since the hydrogel of the present invention can be easily dissolved, cells can be isolated after stimulation for analysis of cell physiology.

Further. the hydrogel of the present invention can be formed having stiffness ranging from 100 Pa to 500 kPa, hence offering a better option to mimic the e.g. stiff natural extracellular matrix of solid tumours. Indeed, such a broad range of stiffness may allow to model tumours at various stages of disease progression.

Hereinafter, the present invention will be described in further detail with reference to examples, but the scope of the present invention will not be construed as being diminished or limited by the examples below.

### EXAMPLES

### Sample illumination

Samples were illuminated with custom-built, microcontroller-regulated illumination panels containing light-emitting diodes (LEDs) with the following manufacturer specifications for the peak wavelengths: 660 nm (LH W5AM, Osram Opto Semiconductors, Regensburg, Germany), 740 nm (LZ4-00R308, LED Engin, San Jose, CA).

### Synthesis of bioink

### Synthesis approach 1: Coupling of PhyB and PIF6 to polymers via Michael-type addition

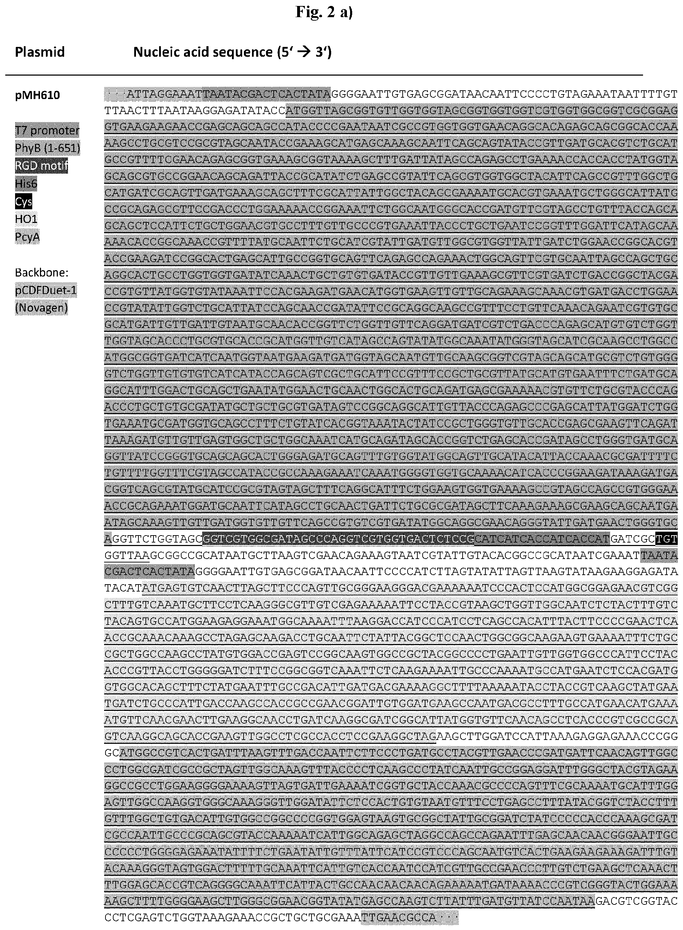

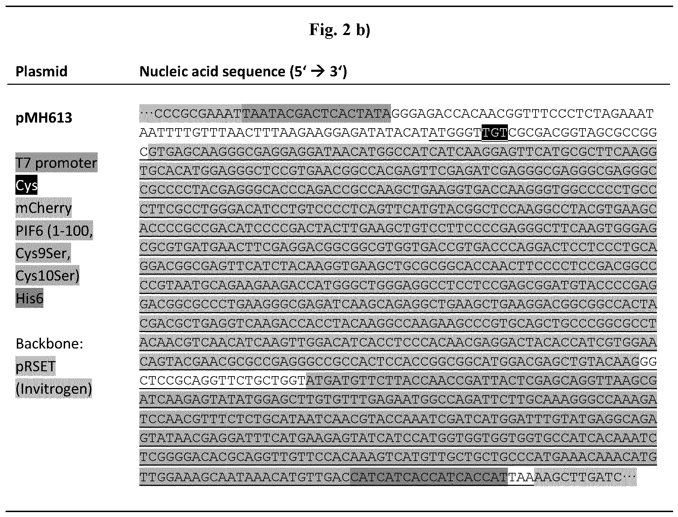

PhyB* (PhyB(*A. thaliana*, amino acids: 1-651)-RGD_motif-His6-Cys) encoded by plasmid pMH610 (SEQ ID NO. 1) (**Fig. 2a**) was produced as holo-protein conjugated to its chromophore phycocyanobilin in E. *coli* by high-cell-density fermentation as described previously (see Horner, M., Gerhardt, K., Salavei, P., Hoess, P., Harrer, D., Kaiser, J., Weber, W. (2019). Production of Phytochromes by High-Cell-Density E. coli Fermentation. ACS Synth. Biol. 8, 2442-2450). PIF6* (Cys-mCherry-PIF6(*A. thaliana*, amino acids: 1-100, Cys9Ser, Cys10Ser)-His6) encoded by plasmid pMH613 (SEQ ID NO. 2) (**Fig. 2b**) was produced in *E. coli* as described previously for GFP-PIF6 (see Beyer, H. M., Thomas, O. S., Riegel, N., Zurbriggen, M. D., Weber, W. & Horner, M. (2018). Generic and reversible opto-trapping of biomolecules. Acta Biomater. 79, 276-282). For protein purification by immobilized metal affinity chromatography (IMAC), bacteria were resuspended in lysis buffer (50 mM NaH₂PO₄, 300 mM NaCl, 10 mM imidazole, pH 8.0) and disrupted by 3 cycles on a high-pressure homogenizer (APV 2000, APV Manufacturing, Bydgoszcz, Poland) at 1,000 bar. After centrifugation at 30,000 x g for 30 min, the clarified lysate was loaded onto a Ni-NTA column (Ni-NTA Superflow, Qiagen, Venlo, Netherlands). Finally, the column was washed with 12 column volumes of wash buffer (50 mM NaH₂PO₄, 300 mM NaCl, 20 mM imidazole, pH 8.0) and the purified protein was eluted with 6 column volumes of elution buffer (50 mM NaH₂PO₄, 300 mM NaCl, 250 mM imidazole, pH 8.0).

Next, the buffer of both proteins was exchanged to hydrogel buffer (PBS (2.7 mM KCl, 1.5 mM KH₂PO₄, 8.1 mM Na₂HPO₄, 137 mM NaCl) with 2 mM EDTA, pH 8.0) by dialysis (regenerated cellulose, 3.5 kDa MWCO) and the proteins were concentrated to ∼50 mg/ml by ultrafiltration (PES membrane, 10 kDa MWCO). Protein concentrations were always determined by Bradford assay (Bio-Rad, Hercules, CA, cat. no. 500-0006) using bovine serum albumin (BSA, Sigma-Aldrich, St. Louis, MO, cat. no. 05479) as standard. Following reduction of the proteins with tris(2-carboxyethyl)phosphine (TCEP, molar ratio of protein : TCEP = 1 : 0.7) for 1 h at room temperature (RT), the proteins were coupled to 8-arm polyethylene glycol-vinylsulfone (PEG-VS, 40 kDa, NOF Europe, Frankfurt, Germany, cat. no. Sunbright HGEO-400VS) at a protein concentration of 30 mg/ml (molar ratio of protein : VS = 3 : 1) in hydrogel buffer supplemented with 100 mM triethanolamine for 18 h at RT. Afterwards, 8-arm PEGs functionalized with PhyB* or PIF6* were purified from uncoupled proteins by size exclusion chromatography (SEC) using a preparative Superdex 200 column (HiLoad 16/600, GE Healthcare, Freiburg, Germany, cat. no. 17-1069-01) and PBS (pH 7.4) as running buffer.

For bioink preparation, purified 8-arm-PEG-PhyB* and 8-arm-PEG-PIF6* were concentrated by ultrafiltration (PES membrane, 100 kDa MWCO) and both functionalized polymers were mixed upon illumination of 8-arm-PEG-PhyB* with near-infrared light in a wavelength range including 740 nm (100 µmol m⁻² s⁻¹, 3 min) in an equimolar ratio (calculated based on PhyB* and PIF6* concentrations) at a PhyB* concentration of 30-60 mg/ml.

### Synthesis approach 2: Coupling of PhyB and PIF6 to polymers via SpyCatcher/SpyTag and SnoopCatcher/SnoopTag reactions

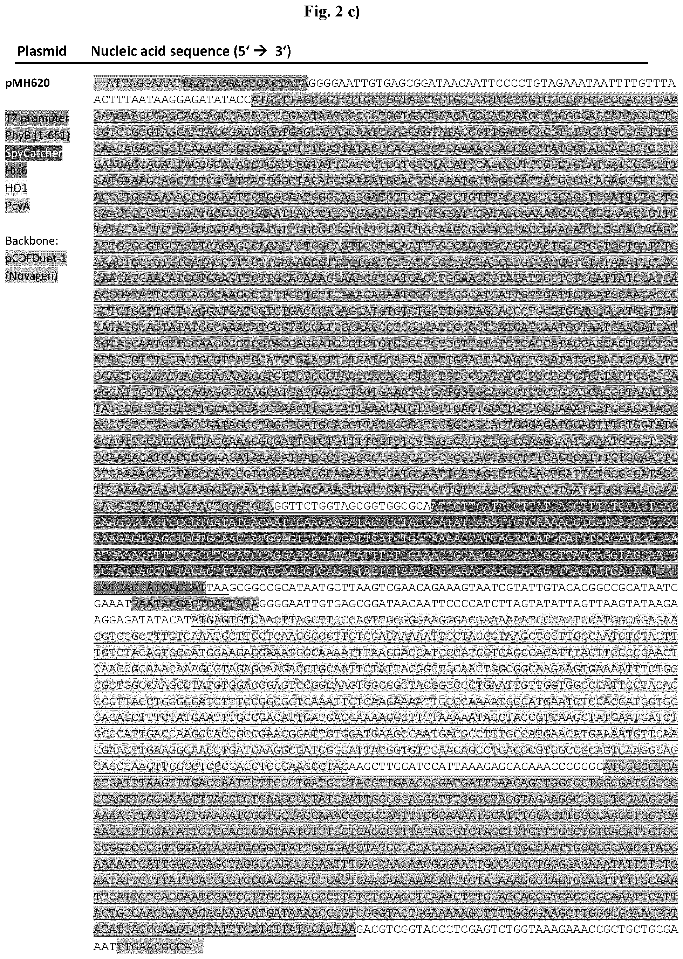

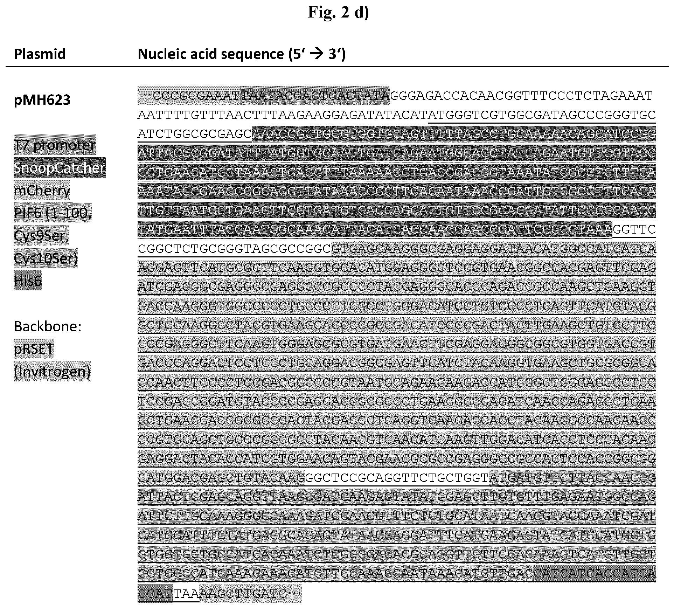

PhyB-SpyCatcher (see Zakeri, B., Fierer, J. O., Celik, E., Chittock, E. C., Schwarz-Linek, U., Moy, V. T. & Howarth, M. (2012). Peptide tag forming a rapid covalent bond to a protein, through engineering a bacterial adhesin. Proc. Natl. Acad. Sci. U.S.A. 109, E690-E697) (PhyB(*A. thaliana*, amino acids: 1-651)-SpyCatcher-His6) encoded by plasmid pMH620 (SEQ ID NO. 3) (**Fig. 2c**) was produced in *E. coli* as described previously for PhyB-AviTag without addition of biotin (Horner, M., Yousefi, O. S., Schamel, W. W. A. & Weber, W. (2020). Production, Purification and Characterization of Recombinant Biotinylated Phytochrome B for Extracellular Optogenetics. Bio Protoc. 10, e3541). PIF6-SnoopCatcher (see Veggiani, G., Nakamura, T., Brenner, M. D., Gayet, R. V., Yan, J., Robinson, C. V. & Howarth, M. (2016). Programmable polyproteams built using twin peptide superglues. Proc. Natl. Acad. Sci. U.S.A. 113, 1202-1207)(SnoopCatcher-mCherry-PIF6(A. *thaliana,* amino acids: 1-100, Cys9Ser, Cys10Ser)-His6) encoded by plasmid pMH623 (SEQ ID NO. 4) (**Fig. 2d**) was produced as described above for PIF6*. Both proteins were purified by IMAC and dialyzed against PBS (pH 7.4) as described above.

To functionalize 8-arm PEG-VS with the SpyTag (see Zakeri et al. cited above) or SnoopTag (see Veggiani et al. cited above) motif, the SpyTag-Cys peptide (AHIVMVDAYKPTKGSGDRCG (N to C terminus)) or SnoopTag-Cys peptide (KLGSIEFIKVNKGSGDRCG (N to C terminus), both synthesized by GenScript, Piscataway, NJ) at a concentration of 20 mg/ml were incubated with 8-arm PEG VS (molar ratio of peptide : VS = 1.5 : 1) in 300 mM triethanolamine (pH 8.0) at RT for 18 h. After removal of uncoupled peptides by SEC as described above, the functionalized PEGs were incubated with the corresponding PhyB-SpyCatcher or PIF6-SnoopCatcher proteins (molar ratio of protein : SpyTag/SnoopTag = 2 : 1) at a protein concentration of 1 mg/ml at RT for 18 h. Following removal of uncoupled proteins by SEC as described above, 8-arm-PEG-SpyTag-PhyB-SpyCatcher and 8-arm-PEG-SnoopTag-PIF6-SnoopCatcher were concentrated by ultrafiltration and the bioink was prepared by mixing both functionalized polymers as described for synthesis approach 1.

### Bioink gelation

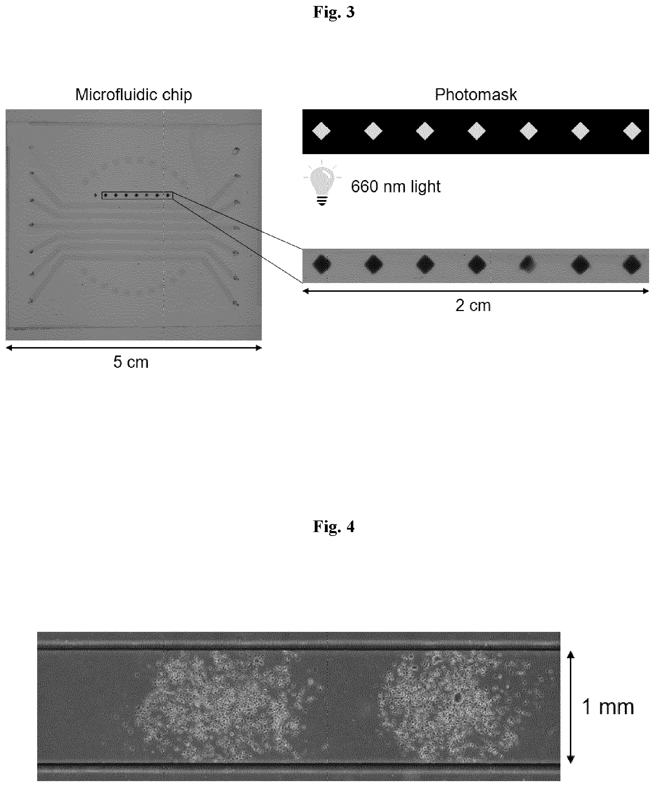

For spatially defined bioink gelation within a channel of a microfluidic chip, 20 µl of the bioink of Synthesis approach 1 with a PhyB* concentration of 46 mg/ml was injected into the channel upon illumination of the bioink with near-infrared light in a wavelength range including 740 nm (100 µmol m⁻² s⁻¹, 3 min). After patterned illumination with visible light in a wavelength range including 660 nm (20 µmol m⁻² s⁻¹, 3 min) using a photomask, non-gelated bioink was removed by flushing the channel with PBS. The set-up of this experiment and experimental results are shown in **Figure 3****.**

### Spatially defined cell deposition

For spatially defined cell deposition within a channel of a microfluidic chip, a bioink of Synthesis approach 1 with a PhyB* concentration of 55 mg/ml and HeLa cells at a concentration of 6,000 cells/µl was injected into a channel of the chip and spatially defined polymerized with visible light in a wavelength range including 660 nm as described above. After flushing the channel with cell culture medium (DMEM (PAN Biotech, Aidenbach, Germany, cat. no. P04-03550), 10 % (v/v) fetal calf serum (FCS, PAN Biotech, cat. no. P30-3602), 100 U ml⁻¹ penicillin, 100 µg ml⁻¹ streptomycin), the hydrogels were dissolved by illumination with near-infrared light in a wavelength range including 740 nm (100 µmol m⁻² s⁻¹, 3 min) and the adherent cells were further incubated at 37 °C and 5 % CO₂. **Figure 4** is an image of the adherent cells in the channel after incubation.

### Bioink characterization

The bioink of Synthesis approach 2 was characterized by a small amplitude oscillatory shear rheology experiment. The small amplitude oscillatory shear experiment was performed on a MCR301 rheometer (Anton Paar, Graz, Austria) with parallel plate configuration. 50 µl of the bioink with a PhyB-SpyCatcher concentration of 46 mg/ml were placed on the lower glass plate (P-PTD 120, Anton Paar, Graz, Austria) of the rheometer and compressed with the upper measuring plate (8 mm diameter, PP08, Anton Paar; gap width: 0.3 mm). Storage and loss moduli were acquired at a deformation of 0.5 % and a frequency of 1 Hz in response to illumination with visible light in a wavelength range including 660 nm (270 µmol m⁻² s⁻¹) or near-infrared light in a wavelength range including 740 nm (180 µmol m⁻² s⁻¹). The hydrogels were illuminated through the glass plate from the bottom and were protected from room light by the temperature chamber (H-PTD 200, Anton Paar). The results are shown in **Figure 5****.**

## Claims

1. A bioink comprising
- a first polymer coupled to a first protein
- a second polymer coupled to a second protein,
wherein the first protein and the second protein form a heterodimer upon irradiation with visible or infrared light in a first wavelength range, which can be reversed upon irradiating the heterodimer with visible or infrared light in a second wavelength range, wherein the second wavelength range is different from the first wavelength range.

2. The bioink of claim 1, wherein the first protein is a phytochrome and the second protein is a phytochrome interacting partner.

3. The bioink of claim 2, wherein the phytochrome is selected from the group comprising PhyA, PhyB, PhyC, PhyD, PhyE and BphP1 and the phytochrome interacting partner is selected from the group comprising PIF1, PIF2, PIF3, PIF4, PIF5, PIF6, PIF7, PIF8, FHY1, FHL, PpsR2 and Q-PAS1.

4. The bioink of any one of claims 1 to 3, wherein the first polymer and the second polymer are coupled to the first protein and the second protein, respectively, via at least one covalent interactions or at least one non-covalent interactions.

5. The bioink of any one of claims 1 to 4, wherein the first polymer and the second polymer are independently selected from the group comprising a polyethylene glycol, a polyacrylamide, a polyacrylate, a poloxamer, a polysaccharide, DNA, polyvinyl alcohol, poly-HEMA, polylysine, polycaprolactone, a protein polymer and a copolymer thereof.

6. The bioink of claim 5, wherein the polyethylene glycol comprises a multiarm polyethylene glycol including 3-arm, 4-arm, 6-arm and 8-arm polyethylene glycol.

7. The bioink of claim 6, wherein the polyethylene glycol has an average molecular weight from 1 kDa to 100 kDa, preferably from 5 kDa to 80 kDa, more preferably from 10 kDa to 60 kDa.

8. The bioink of any one of claims 1 to 7, wherein the first and second polymers are identical.

9. The bioink of any one of claims 1 to 8, wherein the first polymer coupled to the first protein and/or the second polymer coupled to the second protein comprise at least one cell adhesion motif.

10. The bioink of claim 9, wherein the at least one cell adhesion motif is contained in the first protein and/or the second protein.

11. The bioink of claim 9 or 10, wherein the at least one cell adhesion motif is selected from the group consisting of RGD, GFOGER, YGISR and A5G81.

12. A cartridge for a 3D printer, comprising the bioink of any one of claims 1 to 11.

13. A hydrogel, which is formed by irradiating the bioink of any one of claims 1 to 11 with visible or infrared light having a specific wavelength range.

14. A method for reversibly preparing a hydrogel, comprising
(i) preparing a bioink of any one of claims 1 to 11;
(ii) printing the bioink, wherein printing comprises
irradiating the bioink with visible or infrared light in a first wavelength range to obtain a hydrogel.

15. The method of claim 14, further comprising
(iii) irradiating the hydrogel with visible or infrared light in a second wavelength range to dissolve the hydrogel,
wherein the second wavelength range of the light in step (iii) is different from the first wavelength range of the light in step (ii).
